# EUROPEAN PATENT APPLICATION

(11) **EP 4 567 041 A1**
(43) Date of publication of application: **11.06.2025**
(21) Application number: 23214475.8
(22) Date of filing: 06.12.2023
(51) Int. Cl.: C07K 1/32, C07K 14/005, C12P 21/00

(54) **A METHOD FOR THE PURIFICATION OF PROTEINS FUSED TO MULTIPROTEIN COMPLEXES BY METAL AFFINITY PRECIPITATION**

(71) Applicant: Lock and Key Biosciences GmbH, 8952 Schlieren (CH)
(72) Inventor: Garrabou Pi, Xavier, 8706 Meilen (CH); Macdonald, Duncan Stuart, 8057 Zürich (CH)
(74) Representative: Müller-Boré & Partner Patentanwälte PartG mbB

(57) **Abstract**

The present invention relates to a method for the purification of one or more recombinantly expressed protein(s) of interest (POI(s)), comprising the steps of expressing the POI(s) as a fusion protein in which one or more copies of the POI(s) is/are fused to a protomer of a multiprotein complex (MPC), assembling said MPC, said MPC comprising one or more of said fusion protein(s) and any further protomers that are part of said MPC, wherein one or more of the protomers assembled in the MPC contain(s) at least one histidine-rich amino acid sequence, subjecting said MPC in solution to a multi-metal chelate in solution, thereby forming an affinity precipitate, and purifying said affinity precipitate. The present invention further relates to respective affinity precipitates obtained by said method.

## Description

The present invention relates to a method for the purification of one or more recombinantly expressed protein(s) of interest (POI(s)), comprising the steps of expressing the POI(s) as a fusion protein in which one or more copies of the POI(s) is/are fused to a protomer of a multiprotein complex (MPC), assembling said MPC, said MPC comprising one or more of said fusion protein(s) and any further protomers that are part of said MPC, wherein one or more of the protomers assembled in the MPC contain(s) at least one histidine-rich amino acid sequence, subjecting said MPC in solution to a multi-metal chelate in solution, thereby forming an affinity precipitate, and purifying said affinity precipitate. The present invention further relates to respective affinity precipitates obtained by said method.

Recombinant proteins are routinely produced for diverse purposes, including biomedical research, chemical industry, analytic assays, and biotherapeutics. For this end, a host organism is genetically instructed to produce the protein or proteins of interest (POIs) and cultured to the necessary scale. In many instances, once the recombinant protein is expressed, purification procedures are followed to achieve the degree of purity required for specific applications.

Many purification strategies separate the recombinant protein from other molecular entities - which typically include other proteins, nucleic acids, small molecules, and fragments of cellular structures - according to their specific molecular characteristics, such as molecular weight, hydrodynamic volume, electrostatics, or precipitation ability upon addition of certain chaotropic agents. The specific value of the aforementioned properties may vary for each protein of interest, and suitable methods and conditions must be established for each purification target. Alternatively, the recombinant protein can acquire more exclusive properties by merging, at the genetic level, its peptide sequence with a "tag", an additional peptide or protein with affinity for a chemical or biological agent immobilized. Solid supports coated with the bespoken tag binder are used to selectively attach the protein of interest and separate it from other chemical entities in solution. In subsequent steps, the protein-attached solid material is washed with appropriate buffers, and finally the protein of interest is released using a number of strategies, most commonly by addition of a chemical agent that competes with the protein of interest for binding.

One of the most successful strategies for the purification of "tagged" recombinant proteins is Immobilized Metal Affinity Chromatography (IMAC), which typically involves the binding of proteins tagged with a histidine-rich peptide to solid supports derivatized with metal chelates. The most commonly used tag for IMAC is the hexa-histidine, a peptide containing six consecutive histidine residues. Among the metal chelates used for IMAC, molecules containing the chemical structures of nitrilotriacetic acid (NTA) or nitrilodiacetic acid (NDA) have found broad popularity. Both NTA and NDA have high affinity for divalent transition metals such as copper (Cu²⁺), nickel (Ni²⁺), cobalt (Co²⁺), zinc (Zn²⁺), iron (Fe²⁺), and manganese (Mn²⁺). The resulting complexes have a general affinity for surface-exposed, electron-rich residues of proteins, most particularly for histidine residues. Due to entropic effects this affinity is much higher for peptide sequences containing neighbouring histidine residues, as found in the hexa-histidine tag, which provides a highly selective binding for proteins tagged in this way. Specific combinations of histidine residues and other electron rich amino acids attain similar results. Recombinant proteins tagged in this way are readily bonded to IMAC supports, and after washing steps, the bonded proteins are released by addition of a competing weak ligand in high concentrations such as imidazole, by removing the chelated metal with strong chelating agents, such as EDTA, or by a sharp decrease of the pH value that leads to the protonation of histidine residues of the "tag" and renders them unable to bind to metal complexes.

IMAC has become a standard in both research and industrial settings because it is broadly applicable, easy to implement, fast, provides a high degree of purity, and requires minor and typically innocuous changes of the native protein sequence. The technology, however, is not free of setbacks. Solid supports for IMAC are expensive and require thorough cleaning steps to prevent cross-contamination between protein batches. In addition, the solid support used for IMAC is susceptible to unspecific binding of contaminants that may ultimately co-elute with the protein of interest.

Efforts to harness metal affinity without solid support for protein purification have been reported. This approach is very attractive because it relies on affordable chemicals to aggregate and separate the tagged proteins, which reduces costs and prevents cross-contaminations. Precipitation of proteins using bifunctional ligands was first introduced in 1979. In 1989, the first example of protein precipitation based on metal affinity was published, using bis-copper chelates to precipitate proteins with native surface-exposed histidine residues. Subsequently, this concept was combined with the emerging use of hexa-histidine tags to provide more selective precipitation of the protein of interest. In 1991, it was shown that addition of ethylene glycol bis (β-aminoethyl) tetraacetic acid charged with Zn²⁺ [EGTA(Zn)₂] to a solution of a homo-dimeric enzyme tagged with hexa-histidine led to partially selective protein precipitation. The affinity precipitate was washed and subsequently redissolved using EDTA to obtain active enzyme with a certain degree of purification. The protein had been pre-purified using an ammonium sulphate precipitation step. In 1996, metal affinity precipitation was tested on several more polyhistidine-tagged proteins, wherein homo-oligomeric proteins with two or four subunits were precipitated by addition of EGTA(Zn)₂. The purity and yield of the precipitated proteins was very variable, and efficient protein precipitation was only attained at expense of the purity of the collected protein. These meagre results have so far discouraged the extensive use of protein precipitation by metal affinity in spite of its potential benefits.

Thus, there is a need for innovative methods for the metal affinity precipitation of proteins which reliably provide high yields and purity at low cost.

Accordingly, the technical problem underlying the present invention is the provision of improved means for the purification of proteins based on metal affinity precipitation, affording high yields and purities.

The solution to the above technical problem is achieved by the embodiments characterized in the claims.

In particular, in a first aspect, the present invention relates to a method for the purification of one or more recombinantly expressed protein(s) of interest (POI(s)), comprising the steps of:
(a) expressing the POI(s) as a fusion protein in which one or more copies of the POI(s) is/are fused to a protomer of a multiprotein complex (MPC),
(b) assembling said MPC, said MPC comprising one or more of said fusion protein(s) and any further protomers that are part of said MPC,
   wherein one or more of the protomers assembled in the MPC contain(s) at least one histidine-rich amino acid sequence,
(c) subjecting said MPC in solution to a multi-metal chelate in solution, thereby forming an affinity precipitate, and
(d) purifying said affinity precipitate.

The POI(s) to be purified in the present invention do not underlie any particular restrictions and include any proteins the recombinant expression of which might be of interest in any commercial, scientific, or other setting. In specific embodiments, only one POI is expressed. However, in alternative embodiments, two or more, e.g. two, POIs are co-expressed with each other and are assembled in a single MPC. In such embodiments, the POIs may be proteins that are dependent on each other to establish their structure and/or function.

In step (a) of the method of the present invention, the POI(s) is/are expressed as a fusion protein in which one or more copies of the POI(s) is/are fused to a protomer of a multiprotein complex (MPC). Means for recombinantly expressing a respective fusion protein, as well as means for constructing a suitable expression vector, are not particularly limited and are known in the art.

Further, suitable MPC protomers and the respective MPCs are not particularly limited and are known in the art. In preferred embodiments, the MPC contains at least 6 protomers, preferably at least 12 protomers, at least 24 protomers, or at least 48 protomers. Further, in preferred embodiments, at least 6, at least 12, or at least 24 of the protomers assembled in the MPC are identical to each other, i.e., they have the identical amino acid sequence.

In preferred embodiments, the MPC is a symmetric protein assembly, wherein respective symmetric protein assemblies are known in the art. In specific embodiments, the MPC is a protein capsid, preferably wherein the protein capsid is of viral or bacterial origin. In this context, the term "protein capsid" as used herein refers to MPCs that form a porous or non-porous shell encasing an inner lumen. Such protein capsids typically consist of several oligomeric (repeating) structural subunits made of protein called protomers. Protein capsids may display various shapes, such as e.g. spherical or icosahedral. Protein capsids of viral or bacterial origin as used in the present invention can be wild-type protein capsids or modified protein capsids that are merely based on wild-type protein capsids. Suitable modifications, as well as means for modification in this respect, are not particularly limited and are known in the art. Further, computationally designed protein capsids can be used in connection with the present invention, wherein respective means for computational protein capsid design are known in the art.

In particular embodiments, depending on the specific structure of the fusion protein between the POI(s) and the MPC protomer, the POI(s) can be attached to the external surface of the protein capsid, or the POI(s) can be attached to the internal surface of the protein capsid.

In step (b) of the method of the present invention, the MPC is assembled, said MPC comprising one or more of said fusion protein(s) and any further protomers, if any, that are part of said MPC, wherein one or more of the protomers assembled in the MPC contain(s) at least one histidine-rich amino acid sequence. In this context, assembly of an MPC is usually self-assembly of the MPC, i.e., in case all necessary protomers are present in sufficient numbers, the MPC will self-assemble into the MPC. Means for assembling an MPC are not particularly limited and are known in the art. Usually, such means merely encompass co-expression of the required protomers in a host cell and self-assembly of the MPC within the host cell. In specific embodiments, the MPC consists of only one type of protomer, i.e., the fusion protein of the POI(s) fused to the protomer. In other specific embodiments, the MPC consists of said fusion protein and one or more other types of protomer.

According to the present invention, one or more of the protomers assembled in the MPC contain(s) at least one histidine-rich amino acid sequence. In specific embodiments, the fusion protein of the POI(s) fused to the protomer contains said sequence. In other specific embodiments, one or more of the other types of protomer, if any, contain said sequence, either in addition to said fusion protein or instead of said fusion protein.

Histidine-rich sequences that can be used in the context of the present invention are known in the art. These include peptide sequences of 4 to 20 amino acids containing at least three histidine residues. Preferably, the histidine-rich amino acid sequence is selected from the group consisting of the hexahistidine or His-Tag (HHHHHH; SEQ ID NO: 1), the HQ tag (HQHQHQ; SEQ ID NO: 2), the HN tag (HNHNHNHNHNHN; SEQ ID NO: 3), and the HAT tag (KDHLIHNVHKEEHAHAHNK; SEQ ID NO: 4), wherein the His-Tag (HHHHHH; SEQ ID NO: 1) is particularly preferred.

In preferred embodiments, in the fusion protein between the POI(s) and the MPC protomer, the POI(s) is/are fused to the protomer via a linker sequence. Suitable linker sequences are not particularly limited and are known in the art. Preferably, said linker sequence contains a cleavage recognition sequence of a protease. Suitable proteases and their specific cleavage recognition sequences are known in the art. Respective proteases in the proteases can be selected from the group consisting of TEV protease (Tobacco Etch Virus nuclear-inclusion-a endopeptidase), HIV protease (human immunodeficiency virus type 1 protease), HCV NS3 protease (Hepatitis C virus NS3 protease), Factor Xa protease, and thrombin.

In step (c) of the method of the present invention, the MPC, in solution, i.e., in aqueous solution, is subjected to a multi-metal chelate in solution, i.e., in aqueous solution, thereby forming an affinity precipitate. This is effected by the binding of the histidine-rich amino acid sequences present on the MPC to the metal ions present in the multi-metal chelate, thereby linking the MPCs to each other.

Suitable multi-metal chelates are not particularly limited and are known in the art. Preferably, the multi-metal chelate is formed between divalent cations and a complexing organic molecule containing 2 to 6 metal binding sites, wherein said metal binding sites preferably have the molecular formula
linker-N(CH₂CH₂COO⁻)₂, or
linker-NH-(CH₂)ₓ-CH(COO⁻)-N(CH₂COO⁻)₂, wherein x is 2, 3, or 4.

More preferably, said metal binding sites are NTA (nitrilotriacetic acid) or NDA (nitrilodiacetic acid) fragments separated by a linker with at least 4 linearly connected atoms.

The divalent cations are preferably selected from the group consisting of Zn²⁺, Ni²⁺, Co²⁺, Fe²⁺, Cu²⁺, Mn²⁺, and combinations thereof.

In specific embodiments, the multi-metal chelate is a mixture of chelates formed by EGTA (ethylene glycol-bis(β-aminoethyl ether)-N,N,N',N'-tetraacetic acid) and Zn²⁺ (EGTA:Zn).

In the multi-metal chelate used in the present invention, the ratio between the metal ion and the complexing organic molecule is preferably between 1:2 to 2:1.

In preferred embodiments, step (c) of the method of the present invention is performed in an aqueous solution, i.e., both the MPC and the multi-metal chelate are initially solved in said aqueous solution. Further, step (c) of the method of the present invention can be performed in the presence of a weak metal ligand, which can decrease the co-precipitation of contaminants and, thereby, increase purity. Suitable weak metal ligands are not particularly limited and are known in the art. Preferably, the weak metal ligand is imidazole.

The weak metal ligand can be present during step (c) of the method of the present invention at a concentration of 50 to 1000 mM, preferably at a concentration of 100 to 500 mM, more preferably at a concentration of 100 to 200 mM.

In step (d) of the method of the present invention, the affinity precipitate formed in step (c) is purified. Suitable means for the purification are not particularly limited and are known in the art. Preferably, this step (d) is performed by physical means, preferably wherein said physical means are selected from decanting the supernatant from a sedimented suspension of the affinity precipitate, centrifugation, and filtration, as known in the art. Further, step (d) of the method of the present invention can further comprise one or more washing steps, as known in the art, preferably wherein said washing steps comprise one or more cycles of resuspension in an aqueous buffer and/or flushing an aqueous buffer through the affinity precipitate using a filtering device or membrane.

The method of the present invention can further comprise a step of
(e) redissolving the affinity precipitate using a strong metal chelating agent.

Suitable strong metal chelating agents are not particularly limited and are known in the art. Preferably, EDTA is used in this respect.

Further, the method of the present invention can comprise a step of (f) treating the redissolved affinity precipitate with a protease that selectively cleaves a recognition sequence in the linker sequence via which the POI(s) is/are fused to the protomer.

Suitable proteases and cleavage recognition sequences are not particularly limited and are known in the art. Proteases include the proteases selected from the group consisting of TEV protease (Tobacco Etch Virus nuclear-inclusion-a endopeptidase), HIV protease (human immunodeficiency virus type 1 protease), HCV NS3 protease (Hepatitis C virus NS3 protease), Factor Xa protease, and thrombin, as discussed above.

Furthermore, the method of the present invention can comprise a step of
(g) purifying the POI(s),
i.e., purifying the POI(s) after proteolytic cleavage of the affinity precipitate. Suitable means of purifying the POI(s) are not particularly limited and are known in the art.

In a second aspect, the present invention relates to an affinity precipitate obtained by the method of the present invention.

In this aspect, all definitions and limitations provided for the first aspect of the present invention equally apply.

As used herein, the term "comprising"/"comprises" expressly includes the terms "consisting essentially of"/"consists essentially of" and "consisting of"/"consists of", *i.e.,* all of said terms are interchangeable with each other herein.

As used herein, the term "affinity precipitate" refers to the solid obtained by the process of affinity precipitation. Further, the term "affinity precipitation" as used herein refers to the formation of a solid by the interaction, in aqueous solution, of a multi-metal chelate and a multiprotein complex fused to a protein of interest and multiple histidine-rich sequences. The term "cleavage recognition sequence" as used herein refers to an amino acid sequence specifically recognized by a particular protease for hydrolytic cleavage of the peptide chain. The term "EDTA" as used herein refers to ethylenediaminetetraacetic acid and salts thereof, including, but not limited to, ethylenediaminetetraacetic acid disodium salt, ethylenediaminetetraacetic acid disodium salt, ethylenediaminetetraacetic acid trisodium salt, and ethylenediaminetetraacetic acid tetrasodium salt. The term "EGTA" as used herein refers to ethylene glycol-bis(β-aminoethyl ether)-N,N,N',N'-tetraacetic acid and salts thereof, whereas the term "EGTA:Zn" as used herein refers to a mixture of chelates formed by EGTA and Zn²⁺.

Moreover, as used herein, the term "fusion protein" refers to a protein created by merging genetically the sequences of two or more proteins or peptides. The sequences of each of the proteins that constituted the fusion construct are typically spaced by a string of loosely structured amino acids herein named "linker" or "fusion linker" to facilitate the individual folding and the function of each of the proteins merged in the construct.

The term "histidine-rich sequence" as used herein refers to a short peptide sequence of 4 to 20 amino acids containing at least three histidine residues. This definition includes the sequences known in the art to enable protein binding to metal chelates, including, but not limited to, the hexahistidine or His-Tag, (HHHHHH; SEQ ID NO: 1), the HQ tag (HQHQHQ; SEQ ID NO: 2), the HN tag (HNHNHNHNHNHN; SEQ ID NO: 3), and the HAT tag (KDHLIHNVHKEEHAHAHNK; SEQ ID NO: 4).

The term "genetic construct" as used herein refers to a DNA sequence encoding the necessary information for the regulated expression of one or more proteins. In some embodiments, a genetic construct may be supported by one or more plasmids. In some embodiments, a genetic construct may be designed for its insertion into chromosomal DNA of the host organism using homologous recombination techniques.

Further, the term "IMAC" as used herein refers to Immobilized Metal Affinity Chromatography as understood in the art. The term "multi-metal chelates" as used herein refers to the complexes formed between divalent transition metals, including zinc, copper, nickel, cobalt, iron, and manganese, and organic molecules containing at least two NTA or NDA fragments separated by a linker with at least four linearly connected atoms. The ratio between the metal ion and the complexing organic molecule can range between 1:2 to 2:1.

The term "multiprotein complex" as used herein refers to a stable assembly of folded proteins which contains at least six copies of a specific protein. Each of the distinct peptide sequences assembled in a multiprotein complex are defined here as components of a multiprotein complex. In this context, as used herein, the term "protomer" refers to these components of a multiprotein complex, each having a distinct peptide sequence.

As used herein, the term "protease cleavage site" refers to a peptide sequence that is recognized and cleaved with high selectivity by a protease. Further, the term "weak metal ligand" as used herein refers to electron-rich organic molecules that compete with histidine residues for binding to metal chelates. The most commonly used weak metal ligand in the art is imidazole.

The present invention provides methods for the metal affinity precipitation of proteins tagged with a histidine-rich sequence with fast precipitation kinetics, very high recovery, and very high selectivity, upon addition of multi-metal chelates. This innovation relies on two key features.

Firstly, the protein or proteins of interest are expressed as fusion constructs with one or more of the constituents of a multiprotein complex. Suitable multiprotein complexes for this task are, among others, symmetric protein assemblies such as protein capsids from, among others, viral or bacterial origin. The resulting modified multiprotein complex carries up to one of the proteins of interest per associated polypeptide. In addition, a fraction of the peptide sequences associated in the multiprotein complex are tagged with a histidine-rich sequence. As a result, nanoparticles tagged with multiple histidine-rich sequences are formed. In the presence of multi-metal chelates, these nanoparticles bind to each other and aggregate within seconds or minutes. The aggregation efficiency is much higher as compared to oligomeric, hexa-histidine tagged proteins because of the cooperativity effect provided by the binding of a large number of polyhistidine tags. The resulting solid is herein called "affinity precipitate".

Secondly, weak metal ligands, such as imidazole, are known to compete with proteins in the binding of metal chelates. Low concentrations of imidazole, typically 5 to 20 mM, can be used to increase the selective binding of histidine-tagged proteins to solid-immobilized metal chelates and minimize the binding of weaker binders, such as surface exposed histidine residues of other proteins in solution. High concentrations of imidazole (typically 100 to 500 mM) are sufficient to oust histidine-rich sequences from the metal complexes and thereby release proteins from IMAC resins. In some embodiments of the present invention, high concentrations of imidazole (typically 50 to 500 mM) or other weak metal ligands are present during the affinity metal precipitation of the aforementioned multiprotein complexes, which drastically reduces the co-precipitation of contaminants.

In some embodiments of the present invention, the affinity precipitate is subsequently washed with buffers to remove contaminants and exchange the buffer.

Further, in some embodiments of the present invention, the affinity precipitate is used directly in applications harnessing the activity or function of the protein of interest, including, but not limited to, enzymatic activity. This is of particular utility in industrial biocatalysis, in which the re-utilization of the immobilized protein catalyst is crucial to reduce costs.

In some embodiments of the present invention, the affinity precipitate is redissolved partially or totally using a strong chelating agent, such as EDTA.

Further, in some embodiments, the protein of interest is fused to a member of a multiprotein complex through a peptide linker. In some particular embodiments, the peptide linker contains a specific cleavage sequence recognized by a protease. In this case, upon redissolution of the protein affinity precipitate, it is possible to release the protein of interest from the multiprotein complex by hydrolytic cleavage of the linker using a suitable protease.

Furthermore, in some embodiments, the multiprotein complex is a protein capsid. The specific folding and assembly of the protein capsid determines whether the fused proteins of interest are exposed to the surface of the assembled capsid or are encased in the capsid's interior.

The figures show:

### Figure 1:

SDS-PAGE of samples of the purification of Capsid A fused to mCherry, stained with Coomassie Blue. Lane 1: Precision Plus Protein^{™} Unstained Protein Standards, Strep-tagged recombinant (Biorad, 1610363). Lane 2: Sample of full cell lysate. Lane 3: Sample of cleared cell lysate. Lane 4: Sample of supernatant obtained after precipitation of Capsid A/mCherry and separation by centrifugation. Lane 5: Sample of supernatant obtained after first washing step. Lane 6: Sample of supernatant obtained after second washing step. Lane 7: Sample of redissolved Capsid A/mCherry.

### Figure 2:

SDS-PAGE of samples of the purification of Capsid A fused to I-Sce I, stained with Coomassie Blue. Lane 1: Precision Plus Protein^{™} Unstained Protein Standards, Strep-tagged recombinant (Biorad, 1610363). Lane 2: Sample of full cell lysate. Lane 3: Sample of cleared cell lysate. Lane 4: Sample of redissolved Capsid A/I-Sce I.

### Figure 3:

SDS-PAGE of samples of the purification of Capsid A fused to SRSF1, stained with Coomassie Blue. Lane 1: Precision Plus Protein^{™} Unstained Protein Standards, Strep-tagged recombinant (Biorad, 1610363). Lane 2: Sample of full cell lysate. Lane 3: Sample of cleared cell lysate. Lane 4: Sample of supernatant obtained after precipitation of Capsid A/SRSF1 and separation by centrifugation. Lane 5: Sample of supernatant obtained after first washing step. Lane 6: Sample of supernatant obtained after second washing step. Lane 7: Sample of reconstituted Capsid A/SRSF1.

The present invention will be further illustrated by the following examples without being limited thereto.

### Examples

### Example 1:

Capsid A/mCherry was expressed and purified in this example. Capsid A is composed of protomer Alpha, with a hexa-histidine located on a loop exposed to the outside of the capsid structure, and protomer Beta, with a C-terminus pointing to the inside of the capsid and tagged with a linker followed, in this case, by the native sequence of the fluorescent protein mCherry.

Capsid A/mCherry was prepared by co-expression of protomers Alpha and Beta in *E*. *coli* BL21 (DE3) cells. Harvested and frozen cells (5.0 g) were re-suspended in 25 mL of a buffered solution (50 mM NaH₂PO₄, 500 mM NaCl, 100 mM imidazole, pH 8.0) supplemented with Lysozyme (1.0 mg/mL). The suspension was incubated 30 min at 25°C with mild shaking and frozen for 3h at -18°C. The suspension was then melted, and cells were lysed by sonication on ice/water. The lysate was centrifuged (1h, 5000 x g, 4°C) and the supernatant was filtered through a 0.45 micrometer membrane and transferred to a fresh tube on ice.

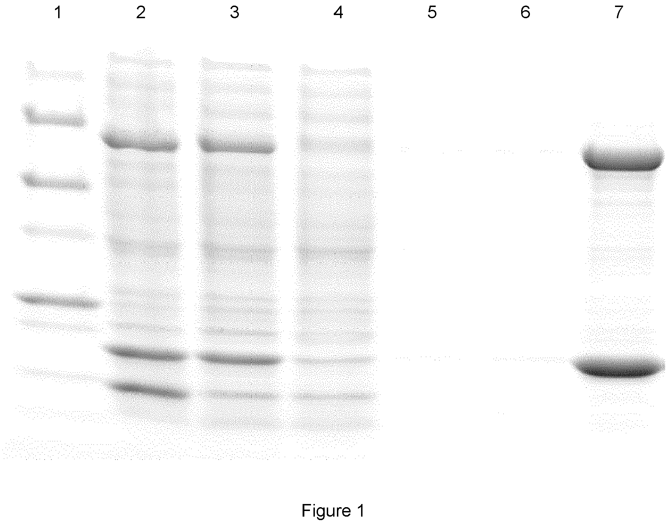

Capsid precipitation was triggered by addition of a 5 mL solution of EGTA-Zn (100 mM EGTA, 150mM ZnCl₂, pH=7.0) and incubation on ice/water for 10 min. The capsid precipitate was collected by centrifugation and the supernatant was discarded. More than 90% of the soluble capsid was precipitated as determined by color densitometry analysis of a Coomassie-stained SDS-PAGE (Fig. 1). The capsid precipitate was resuspended in 5 mL of a washing buffer (50 mM NaH₂PO₄, 500 mM NaCl, pH 8.0) and collected again by centrifugation, discarding the supernatant. The washing step was repeated, and the washed precipitate was redissolved in 1 mL of a solution (50 mM NaH₂PO₄, 500 mM NaCl, pH 8.0) containing 50 mM EDTA. The purity of the redissolved capsid was >90% as determined by color densitometry analysis of a Coomassie-stained SDS-PAGE (Fig. 1).

### Example 2:

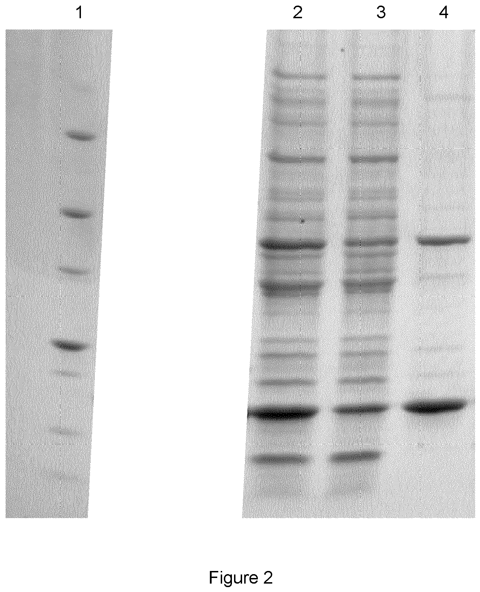

Capsid A/I-Sce I was expressed and purified in this example. The procedure of Example 1 was followed, using the gene encoding the Intron-encoded endonuclease (I-Sce I) from *Saccharomyces cerevisiae* instead of mCherry. The solution obtained after the purification procedure contained Capsid A/I-Sce I in a purity >90% as determined by color densitometry analysis of a Coomassie-stained SDS-PAGE (Fig. 2).

### Example 3:

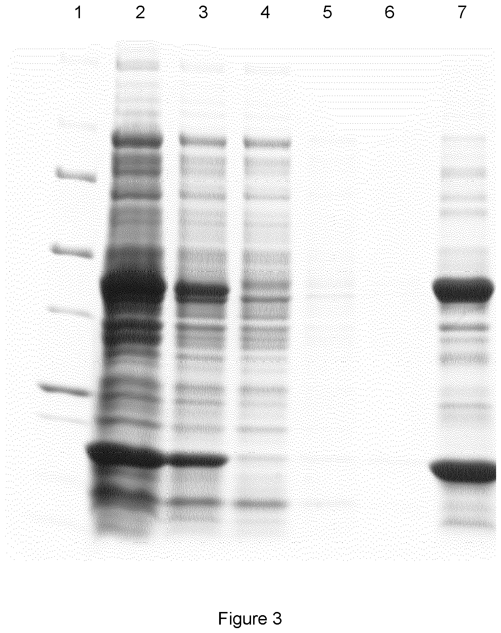

Capsid A/SRSF1 was expressed and purified in this example. The procedure of Example 1 was followed, using the gene encoding human Serine/arginine-rich splicing factor 1 (SRSF1) instead of mCherry. More than 90% of the soluble capsid was precipitated as determined by color densitometry analysis of a Coomassie-stained SDS-PAGE (Fig. 3). The solution obtained after the purification procedure contained Capsid A/SRSF1 in a purity >90% as determined by color densitometry analysis of a Coomassie-stained SDS-PAGE (Fig. 3).

### Example 4:

Capsid B/mCherry was expressed and purified in this example. Capsid B is composed of protomer Alpha, with a C-terminal hexa-histidine exposed to the outside of the capsid structure, and protomer Beta, with a C-terminus pointing to the inside of the capsid and tagged with a linker followed, in this case, by the native sequence of the fluorescent protein mCherry.

Capsid B/mCherry was prepared by co-expression of protomers Alpha and Beta in *E*. *coli* BL21 (DE3) cells. Harvested and frozen cells (5.0 g) were re-suspended in 25 mL of a buffered solution (50 mM NaH₂PO₄, 500 mM NaCl, 100 mM imidazole, pH 8.0) supplemented with Lysozyme (1.0 mg/mL). The suspension was incubated 30 min at 25°C with mild shaking and frozen for 3h at -18°C. The suspension was then melted, and cells were lysed by sonication on ice/water. The lysate was centrifuged (1h, 5000 x g, 4°C) and the supernatant was filtered through a 0.45 micrometer membrane and transferred to a fresh tube on ice. Capsid precipitation was triggered by addition of a 5 mL solution of EGTA-Zn (100 mM EGTA, 150mM ZnCl₂, pH=7.0) and incubation on ice/water for 10 min. The capsid precipitate was collected by centrifugation and the supernatant was discarded.

More than 90% of the soluble capsid was precipitated as determined by color densitometry analysis of a Coomassie-stained SDS-PAGE (not shown). The capsid precipitate was resuspended in 5 mL of a washing buffer (50 mM NaH₂PO₄, 500 mM NaCl, pH 8.0) and collected again by centrifugation, discarding the supernatant. The washing step was repeated, and the washed precipitate was redissolved in 1 mL of a solution (50 mM NaH₂PO₄, 500 mM NaCl, pH 8.0) containing 50 mM EDTA. The purity of the redissolved capsid was >90% as determined by color densitometry analysis of a Coomassie-stained SDS-PAGE (not shown).

## Claims

1. A method for the purification of one or more recombinantly expressed protein(s) of interest (POI(s)), comprising the steps of:
(a) expressing the POI(s) as a fusion protein in which one or more copies of the POI(s) is/are fused to a protomer of a multiprotein complex (MPC),
(b) assembling said MPC, said MPC comprising one or more of said fusion protein(s) and any further protomers that are part of said MPC, wherein one or more of the protomers assembled in the MPC contain(s) at least one histidine-rich amino acid sequence,
(c) subjecting said MPC in solution to a multi-metal chelate in solution, thereby forming an affinity precipitate, and
(d) purifying said affinity precipitate.

2. The method of claim 1, wherein the histidine-rich sequence is a peptide sequence of 4 to 20 amino acids containing at least three histidine residues, preferably wherein the histidine-rich amino acid sequence is selected from the group consisting of the hexahistidine or His-Tag (HHHHHH; SEQ ID NO: 1), the HQ tag (HQHQHQ; SEQ ID NO: 2), the HN tag (HNHNHNHNHNHN; SEQ ID NO: 3), and the HAT tag (KDHLIHNVHKEEHAHAHNK; SEQ ID NO: 4),
more preferably wherein the histidine-rich amino acid sequence is a His-Tag (HHHHHH; SEQ ID NO: 1).

3. The method of claim 1 or claim 2, wherein in said fusion protein, the POI(s) is/are fused to the protomer via a linker sequence,
preferably wherein said linker sequence contains a cleavage recognition sequence of a protease,
preferably wherein said protease is selected from the group consisting of TEV protease (Tobacco Etch Virus nuclear-inclusion-a endopeptidase), HIV protease (human immunodeficiency virus type 1 protease), HCV NS3 protease (Hepatitis C virus NS3 protease), Factor Xa protease, and thrombin.

4. The method of any one of claims 1 to 3, wherein the MPC is a symmetric protein assembly,
preferably wherein the MPC is a protein capsid,
preferably wherein the protein capsid is of viral or bacterial origin.

5. The method of claim 4, wherein the POI(s) is/are attached to the external surface of the protein capsid or to the internal surface of the protein capsid.

6. The method of any one of claims 1 to 5, wherein the multi-metal chelate is formed between divalent cations and a complexing organic molecule containing 2 to 6 metal binding sites,
preferably wherein said metal binding sites have the molecular formula
linker-N(CH₂CH₂COO⁻)₂, or
linker-NH-(CH₂)ₓ-CH(COO⁻)-N(CH₂COO⁻)₂, wherein x is 2, 3, or 4, more preferably wherein said metal binding sites are NTA (nitrilotriacetic acid) or NDA (nitrilodiacetic acid) fragments separated by a linker with at least 4 linearly connected atoms.

7. The method of claim 6, wherein said divalent cations are selected from the group consisting of Zn²⁺, Ni²⁺, Co²⁺, Fe²⁺, Cu²⁺, Mn²⁺, and combinations thereof.

8. The method of any one of claims 1 to 7, wherein the multi-metal chelate is a mixture of chelates formed by EGTA (ethylene glycol-bis(β-aminoethyl ether)-N,N,N',N'-tetraacetic acid) and Zn²⁺ (EGTA:Zn).

9. The method of any one of claims 1 to 8, wherein in the multi-metal chelate, the ratio between the metal ion and the complexing organic molecule is between 1:2 to 2:1.

10. The method of any one of claims 1 to 9, wherein step (c) is performed in an aqueous solution.

11. The method of any one of claims 1 to 10, wherein step (c) is performed in the presence of a weak metal ligand,
preferably wherein said weak metal ligand is imidazole,
preferably wherein said weak metal ligand is present at a concentration of 50 to 1000 mM.

12. The method of any one of claims 1 to 11, further comprising the step:
(e) redissolving the affinity precipitate using a strong metal chelating agent, preferably wherein said strong metal chelating agent is EDTA (ethylenediaminetetraacetic acid).

13. The method of any one of claims 1 to 12, further comprising the step:
(f) treating the redissolved affinity precipitate with a protease that selectively cleaves a recognition sequence in the linker sequence via which the POI(s) is/are fused to the protomer.

14. The method of any one of claims 1 to 13, further comprising the step:
(g) purifying the POI(s).

15. An affinity precipitate obtained by the method of any one of claims 1 to 14.
